# EUROPEAN PATENT SPECIFICATION

(11) **EP 1 503 655 B1**
(45) Date of publication and mention of the grant of the patent: **08.07.2009**
(21) Application number: 03722645.3
(22) Date of filing: 05.05.2003
(51) Int. Cl.: A61B 3/10

(54) **VISUAL STIMULATOR**
VISUELLER STIMULATOR
STIMULATEUR VISUEL

(30) Priority: 06.05.2002 FI 20020851
(43) Date of publication of application: 09.02.2005
(73) Proprietor: Valjakka, Antti, 71570 Syvänniemi (FI)
(72) Inventor: Valjakka.Antti, 71570 Syvänniemi (FI); Urtti, Arto, 70420 Kuopio (FI); Ahonen, Janne, 70260 Kuopio (FI)
(74) Representative: Pitkänen, Hannu Alpo Antero
(86) International application number: PCT/FI2003/000351
(87) International publication number: WO 2003/092483

(56) References cited:
- WO-A1-01/78586
- WO-A1-02/41768
- DE-A1- 4 301 483

## Description

The present invention relates to a visual stimulator in accordance with the preamble of claim 1.

There are two technical requirements in stimulating techniques applied in visual measurement methods for creating a visual response: the way the visual stimulus has been pointed to the eye and the way other factors of the stimulus source than factors of light effect have been eliminated from the object for measurement. In visual measurement methods based on recognized techniques which have been designed for measuring the physiological potential change of retina of the eye (electroretinogram, ERG) for a visual stimulus carried out on test animals has been represented that by applying mirrors, lenses or a LED-light source or a flashlight aligned to the eye the beams of the light may be pointed to the retina and to various parts of it while the subject to be examined stays on place or has by some means made immovable. (Documenta Ophthalmologica 98, 2000, Bayer A.U., Mittag T., Cook P., Brodie S.E., Podos S.M. and Maag K-P. "Comparisons of the amplitude size and the reproducibility of three different electrodes to recorded the corneal flash electroretinogram in rodents", p. 233-246; Documenta Ophthalmalogica 81, 1992, Hawlina M. and Konec B., "New noncomeal HK-loop electrode for clinical electroretinography", p. 253-259; US-patent 4 255 023; US-patent 5 506 633; SU-patent publication 1 648 336).

The measuring of ERG-potentials corresponding with the magnitude of visual stimulus from a laboratory animal or a human moving freely creates a technical problem because in free motion the direction of gaze in 3-dimensional space as well as the place of the body or a part of it such as the head continuously changes between different measurements. Another common technical problem while applying some visual stimulus sources, such as a flashlight, are other physical effects created by them than the changes in light intensity, which other physical effects may be the change in magnetic field and sound, among other things. The solving of the problem should effect such that the magnitude of the visual sense is independent of the direction of the gaze of the subject, the body or a part of it may not hinder the transmitting of the visual stimulus and other than light effects of the light stimulus source are excluded in the object for measuring.

The object of the invention is to eliminate these disadvantages and to provide a visual stimulator, which enables the registration of an ERG-response created by a visual stimulus as well as registration of other biopotential responses connected with other visual processes from a laboratory animal or a human moving freely and being conscious such that the direction of the gaze or the place of the body or a part of the body, such as the head, must not be constant between different measuring situations. In addition, the object of the invention is to provide a device which excludes other than light stimulating factors, which other factors might arise from the applying of the light stimulating source.

The object of the invention is accomplished by a visual stimulator, the characteristics of which are presented in claim 1.

The visual stimulator in accordance with the invention comprises a mirror chamber and a mirror tube constructed of mirrors, in which chamber and tube the reflecting sites of the mirrors form various angles with respect to each other, and which sites have been connected to each other such that light beams are dispersed from the mirror tube to the mirror chamber without hindrance regardless of the location of the object or a part of an object to be examined in the mirror chamber, and to the other end of the mirror tube, as seen away from the mirror chamber, a light stimulating source has been aligned.

The above mentioned purposes are achieved by applying a visual stimulator in accordance with the invention, which stimulator comprises a mirror chamber and a mirror tube, which have been connected to each other such that while a laboratory animal to be examined, or some other biological object such as the head of a human has been placed in the mirror chamber light beams are dispersed from the mirror tube to the mirror chamber without hindrance regardless of the location of the object or a part of an object to be examined in the mirror chamber. An even dispersion of light beams in the mirror chamber is created by parts of the mirror surfaces of the mirror chamber and mirror tube located in various angles with respect to each other, while the magnitude of the exposition of the object for a visual stimulus is independent of the change of the direction of the gaze, which may be caused by eye movements or the change in place of the body. Light is reflected from a light stimulus source, such as a flashlight, to disperse to a closed mirror tube the inner surfaces of which are reflecting and through it such that the distance that the mirror tube forms from light stimulus source to the mirror chamber, in its part, excludes other factors than the desired change in light intensity in the mirror chamber. A change in magnetic field and sound while applying a flashlight may be, for example, these kind of non-light effecting factors caused by light stimulus source.

While more than one light stimulating source are applied in the solution in accordance with the invention presented above the dynamic function of visual sense may be measured for visual stimuli presented in variable time sequences, which visual stimuli may be discrete or in various ways cumulatively overlapping with respect to time and intensity.

In addition, while the photon flow of each visual stimulus, which may be a light flash of short duration, is selectable regulable according to its wave length it is possible to modulate the spectrum of factors having influence on the visual stimulation as desired: the sequence or state of cumulation in time of the stimulation, the chromatic characteristics of the stimulation and the intensity of the stimulation and such measure the general dynamics of sight.

Next, the invention will be explained in more detail with reference to the accompanying drawings, in which,
Figure 1 illustrates a visual stimulator in accordance with the invention viewed from above,
Figure 2 illustrates a visual stimulator in accordance with figure 1 viewed from side,
Figure 3 illustrates parts of a visual stimulator in accordance with figure 1 viewed separately inclined from side,
Figure 4 illustrates the path of a light beam in a visual stimulator in accordance with figure 1, and
Figure 5 illustrates the path of a light beam in a visual stimulator in accordance with figure 2.

The visual stimulator in accordance with the figures comprises a mirror chamber 1 constructed of one or several mirrors 6 and a mirror tube 2 constructed of one or several mirrors 14 and which tube is connected to the chamber. In addition, the visual stimulator comprises a light stimulation source 4 placed in the other end of the mirror tube, an attenuator device 3 as well as photodetectors 11 and 18.

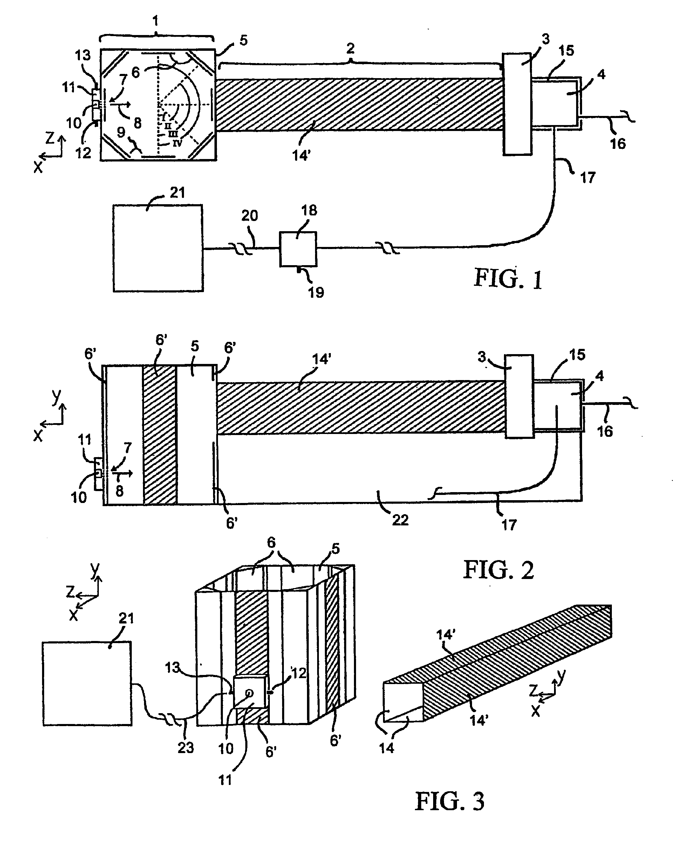

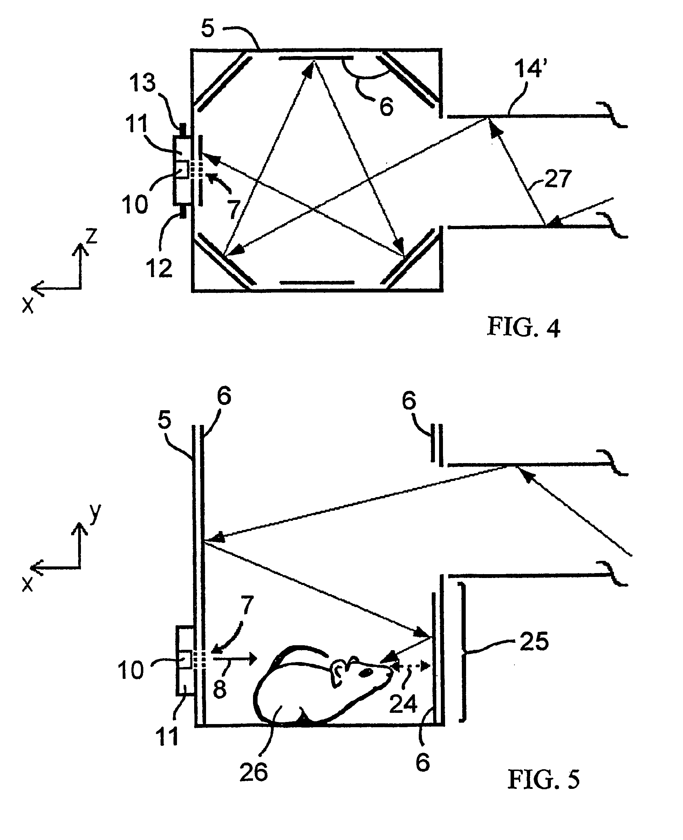

The mirror chamber 1 comprises an outer wall 5, which has been made of transparent material in this application, such as Lexan-plastic, advantageously, or corresponding or glass, and it gives limits to a square bottom area forming a tetrahedron left open in one end. The bottom of the tetrahedron may be constructed removable for cleaning possibility. On the inner surface of the chamber 1, in symmetric order viewed from above, mirrors 6 have been adjusted such that there are gaps 9, illustrated in figure 1, left between the mirrors for observing the space for a laboratory animal 26 in accordance with figures 4 and 5. The reflecting surface 6 of the mirrors is facing inside the chamber 1. The mirrors 6 in the mirror chamber 1 form various angles with respect to each other (angles I, II, III, IV in figure 1 for example). In this application the mirrors 6 are planelike and placed such that the angle I equals 45°, the angle II 90°, the angle III 135° and the angle IV 180°. In the other application mirrors may be unlimited curved. The silver surface 6' of all mirrors has been earthened to the same 0-potential of the system.

Against one outer wall, viewed from outside, of the mirror chamber 1 a photodetector 11 has been adjusted such that the observing line 8 of the sensor 10 in it is directed through an opening 7 placed on the outer wall 5 and in the mirror 6 to the mirror chamber 1 such that the photodetector 11 observes the light intensity in the mirror chamber 1. The sensor 10 of the photodetector 11 has been placed in vertical direction to correspond with the horizontal level 24 of the visual field of the laboratory animal 26. The voltage pulse describing the intensity of light measured by the photodetector 11 is transmitted from the outlet connector 13 with a conductor 23 to the automatic data processing device 21 illustrated in figures 1 and 3 for registration and storing, where the intensity of light as a function in time may be determined. The photodetector 11 may employ external voltage as its power source, which voltage is transmitted to the connector 12. There may be more than one photodetector in the mirror chamber.

The mirror tube 2 open in the ends and square by cross-section has been directed against one outer wall of the mirror chamber 1 viewed from outside. The reflecting surfaces 14 of the mirrors of the mirror tube form the inner wall of the tube 2. In the connection point of the mirror tube 2 and the mirror chamber 1 on the wall of the mirror chamber 1 there is an opening corresponding with the cross-sectional area of the mirror tube 2 or optionally at this point on the inner surface of the outer wall 5 of the mirror chamber 1 there are no mirrors. The mirror tube 2 has been supported on a stand 22 in accordance with figure 2 on the height 25 where the body or a part of the body of a laboratory animal 26 may not hinder the dispersion of light beams from the mirror tube 2 to the mirror chamber 1. The silver surface 14' of all mirrors of the mirror tube has been earthened to the same 0-potential of the system. The mirrors 14 in this application are planelike mirrors. In the other application the mirrors may be unlimited curved.

In the end of the mirror tube, viewed away from the mirror chamber, there is the light stimulation source 4, the light reflecting surface of which has been directed to the mirror tube 2. The light stimulation source 4 has been covered with metal protection cover 15, excluding the surface reflecting light, which cover has been earthened to the 0-potential of the system. The metal protection cover has been manufactured, advantageously, of a metal plate of sufficient thickness (about 2 mm for example), in order, for its part, to eliminate the influence of the magnetic field and sound created by the light stimulation source 4 on the measuring system and the subject 26 to be examined in the mirror chamber 1. The light stimulation source 4 may be activated either with a switch on it, electronically with a conductor 16 or wirelessly with a remote control, the field pulse of which is such that it has no influence on other parts of the device than on the light stimulation source 4 to be controlled. Light beams 27 reflect from the light stimulation source 4 through a regulated attenuator 3 placed between the light stimulation source 4 and the mirror tube 2 to the mirror tube 2, where they are dispersed and from where they continue further to the mirror chamber 1.

The attenuator 3 has been organized to attenuate various wavelengths of light with optional efficiencies. In the other application the attenuator of the light intensity has been placed between the mirror tube and the mirror chamber.

There is a fibre optics cable 17 connected to the light stimulation source 4, which fibre optics cable conducts light created by the light stimulus source 4 to the photodetector 18, where from the information about the intensity of light is transmitted through an electronic conductor 21 to be registered and stored. The optic isolation technique described enables comparing of light intensity of the light stimulation source 4 with the induced ERG-response as a function of time as well as the fact that the biopotential signal is free of disturbance, which could be transmitted from conductors in case they were connected to the light stimulation source 4 or near to it.

Photodetectors 11 and 18 comprise a phototransistor and/or a photodiode as well as an operation amplifier amplifying the light signal. Silver surfaces 6' and 14' of all mirrors 6 and 14 of the device as well as the metal protection cover 15 have been galvanically connected to each other.

In accordance with what above has been presented the biological object placed in the mirror chamber may be only a head of a subject, of a human, for example. The chamber may be lowered down by means of supporting rods round the sitting test subject's head. In this case it is practical that the mirror tubes overlap mutually into spiral curves (see claims).

In accordance with the idea of the invention the mirror tube 2 is employed as focusing device of light beams from the light stimulation source 4 to that point of the mirror chamber 1 where the subject 26 may not form a hindrance for light dispersion from the mirror tube 2 to the mirror chamber 1. The mirror tube 2 enables the light reflection to the mirror chamber 1 from such a distance that it, for its part, eliminates other effects (change in magnetic field, sound stimulus) of the light stimulation source 4 than changes of the light intensity in the mirror chamber 1. The mirror chamber 1 functions as a space where a laboratory animal 26 may freely move around in a limited area and where light beams disperse evenly in 3-dimensional space when the mirror chamber 1 as well as the mirror tube 2 connected to it have been made of mirrors 6 and 14, which mirrors or their reflecting parts form various angles with respect to each other.

Within the limits of the invention also other than above described solution may be considered. Such, the mirror tube may be of any shape and of any size, it may be made of one or several mirrors and it or those may be directed to the mirror chamber from different directions such, however, that the principle stays as presented. For example, a conical mirror tube may be directed vertically to the upper part of the mirror chamber. The number of mirror tubes or light stimulation sources is not limited. In the same way the mirror chamber in accordance with the presented principle may be of any shape and size and it may be made of one or several mirrors. As a light stimulation source a glow bulb or some other recognized source of light instead of a flashlight may be applied.

The invention is not limited to the presented advantageous application but it can vary within the frames of the idea of the invention formed in the claims.

## Claims

1. A visual stimulator, which comprises a light stimulation source (4), a regulable attenuator of light (3), mirrors (6 and 14) for reflecting light and light detectors (11 and 18), **characterized in that** the visual stimulator comprises a mirror chamber (1) and a mirror tube (2) constructed of mirrors (6 and 14) in which the reflecting points of the mirrors (6 and 14) form various angles with respect to each other, and which are connected to each other such that light beams (27) disperse from the mirror tube (2) to the mirror chamber (1) without hindrance regardless of the location in the mirror chamber of the object to be examined (26), and that the end of the mirror tube (2) opposite from the end to which the mirror chamber is connected is equipped with the light the stimulation source (4).

2. A visual stimulator in accordance with claim 1, **characterized in that** there is one or several mirrors (6) in the mirror chamber (1).

3. A visual stimulator in accordance with claim 1 and 2, **characterized in that** the mirror (6) of the mirror chamber (1) is planelike.

4. A visual stimulator in accordance with claim 1, **characterized in that** there is one or several mirrors (14) in the mirror tube (2).

5. A visual stimulator in accordance with claims 1 and 4, **characterized in that** the mirror (14) of the mirror tube (2) is planelike.

6. A visual stimulator in accordance with claim 1, **characterized in that** the outer wall (5) of the mirror chamber (1) is made of a transparent material.

7. A visual stimulator in accordance with claims 1 and 6, **characterized in that** the outer wall (5) of the mirror chamber (1) is made of plastic or glass.

8. A visual stimulator in accordance with claims 1, 2 and 7, **characterized in that** there are openings (9) between the mirrors (6) of the mirror chamber (1).

9. A visual stimulator in accordance with claim 1, **characterized in that** the regulable attenuator of light (3) is placed between the light stimulation source (4) and the mirror tube (2).

10. A visual stimulator in accordance with claim 1, **characterized in that** the regulable attenuator of light (3) is placed between the mirror tube (2) and the mirror chamber (1).

11. A visual stimulator in accordance with claim 1, **characterized in that** more than one light stimulation source (4) is directed to the mirror tube (2).

12. A visual stimulator in accordance with claim 1, **characterized in that** the light stimulation source (4) is a flashlight.

13. A visual stimulator in accordance with claim 1, **characterized in that** the light stimulation source (4) is covered with metal protection cover (15) excluding its light reflecting surface.

14. A visual stimulator in accordance with claim 1, **characterized in that** a fibre optics cable (17) is connected to the light stimulation source (4), which cable is connected to the light detector (18) from where the information about light intensity is conveyable to the data processing device (21).

15. A visual stimulator in accordance with claim 1, **characterized in that** the mirror chamber (1) comprises one or several light detectors (11) from which the information about light intensity is conveyable to the data processing device (21).

16. A visual stimulator in accordance with claims 1, 14 and 15, **characterized in that** the light detectors (11 and 18) comprise a phototransistor and/or a photodiode as well as an operation amplifier amplifying the light signal.

17. A visual stimulator in accordance with claims 1, 2, 3, 4, 5, and 13, **characterized in that** silver surfaces (6' and 14') of the mirrors (6 and 14) of the device as well as the metal protection cover (15) of the light stimulation source (4) are galvanically connected to each other.

18. A visual stimulator in accordance with claim 1, **characterized in that** the regulable attenuator of light (3) is derised to attenuate various wavelengths of light with selectable efficiency.

## Patentansprüche

1. Visueller Stimulator, welcher eine Licht-Stimulationsquelle (4), einen regulierbaren Lichtabschwächer (3), Spiegel (6 und 4) zum Reflektieren von Licht und Lichtdetektoren (11 und 18) aufweist, **dadurch gekennzeichnet, dass** der visuelle Stimulator eine Spiegelkammer (1) und eine Spiegelröhre (2) aufweist, die aus Spiegeln (6 und 14) ausgebildet ist, in welcher die reflektierenden Punkte der Spiegel (6 und 14) unterschiedliche Winkel mit Bezug zueinander bilden, und welche miteinander verbunden sind, so dass sich Lichtstrahlen (27) von der Spiegelröhre (2) zu der Spiegelkammer (1) ohne Hindernis ausbreiten, ungeachtet der Position der Spiegelkammer des Objektes (26), welches untersucht werden soll, und dass das Ende der Spiegelröhre (2) gegenüber von dem Ende, mit welchem die Spiegelkammer verbunden ist, mit der Licht-Stimulationsquelle (4) ausgestattet ist.

2. Visueller Stimulator in Übereinstimmung mit Anspruch 1, **dadurch gekennzeichnet, dass** es einen oder mehrere Spiegel (6) in der Spiegelkammer (1) gibt.

3. Visueller Stimulator in Übereinstimmung mit Anspruch 1 und 2, **dadurch gekennzeichnet, dass** der Spiegel (6) der Spiegelkammer (1) flächenförmig ist.

4. Visueller Stimulator in Übereinstimmung mit Anspruch 1, **dadurch gekennzeichnet, dass** es einen oder mehrere Spiegel (14) in der Spiegelröhre (2) gibt.

5. Visueller Stimulator in Übereinstimmung mit Anspruch 1 und 4, **dadurch gekennzeichnet, dass** der Spiegel (14) der Spiegelröhre (2) flächenförmig ist.

6. Visueller Stimulator in Übereinstimmung mit Anspruch 1, **dadurch gekennzeichnet, dass** die äußere Wand (5) der Spiegelkammer (1) aus einem transparenten Material gefertigt ist.

7. Visueller Stimulator in Übereinstimmung mit den Ansprüchen 1 und 6, **dadurch gekennzeichnet, dass** die äußere Wand (5) der Spiegelkammer (1) aus Plastik oder Glas gefertigt ist.

8. Visueller Stimulator in Übereinstimmung mit den Ansprüchen 1, 2 und 7, **dadurch gekennzeichnet, dass** es zwischen den Spiegeln (6) der Spiegelkammer ( 1 ) Öffnungen (9) gibt.

9. Visueller Stimulator in Übereinstimmung mit Anspruch 1, **dadurch gekennzeichnet, dass** ein regulierbarer Lichtabschwächer (3) zwischen der Licht-Stimulationsquelle (4) und der Spiegelröhre (2) angeordnet ist.

10. Visueller Stimulator in Übereinstimmung mit Anspruch 1, **dadurch gekennzeichnet, dass** ein regulierbarer Lichtabschwächer (3) zwischen der Spiegelröhre (2) und der Spiegelkammer (1) angeordnet ist.

11. Visueller Stimulator in Übereinstimmung mit Anspruch 1, **dadurch gekennzeichnet, dass** mehr als eine Licht-Stimulationsquelle (4) auf die Spiegelröhre (2) gerichtet ist.

12. Visueller Stimulator in Übereinstimmung mit Anspruch 1, **dadurch gekennzeichnet, dass** die Licht-Stimulationsquelle (4) ein Blitzlicht ist.

13. Visueller Stimulator in Übereinstimmung mit Anspruch 1, **dadurch gekennzeichnet, dass** die Licht-Stimulationsquelle (4) mit einer Metall-Schutzabdeckung (15) abgedeckt ist, welche ihre lichtreflektierende Oberfläche ausschließt.

14. Visueller Stimulator in Übereinstimmung mit Anspruch 1, **dadurch gekennzeichnet, dass** ein faseroptisches Kabel (17) mit der Licht-Stimulationsquelle (4) verbunden ist, wobei das Kabel mit dem Lichtdetektor (18) verbunden ist, von welchem die Information über eine Lichtintensität an eine Datenverarbeitungseinrichtung (21) übertragbar ist.

15. Visueller Stimulator in Übereinstimmung mit Anspruch 1, **dadurch gekennzeichnet, dass** die Spiegelkammer (1) einen oder mehrere Lichtdetektoren (11) aufweist, von welchen die Information über eine Lichtintensität an die Datenverarbeitungseinrichtung (21) übertragbar ist.

16. Visueller Stimulator in Übereinstimmung mit den Ansprüchen 1, 14 und 15, **dadurch gekennzeichnet, dass** die Lichtdetektoren (11 und 18) einen Phototransistor und/oder eine Photodiode, wie auch einen Operationsverstärker aufweisen, welcher das Lichtsignal verstärkt.

17. Visueller Stimulator in Übereinstimmung mit den Ansprüchen 1, 2, 3, 4, 5 und 13, **dadurch gekennzeichnet, dass** Silberoberflächen (6' und 14') der Spiegel (6 und 14) der Einrichtung, wie auch die Metall-Schutzabdeckung (15) der Licht-Stimulationsquelle (4), galvanisch miteinander verbunden sind.

18. Visueller Stimulator in Übereinstimmung mit Anspruch 1, **dadurch gekennzeichnet, dass** der regulierbare Lichtabschwächer (3) erwünscht ist, um unterschiedliche Wellenlängen von Licht mit auswählbarer Effizienz abzuschwächen.

## Revendications

1. Stimulateur visuel, comprenant une source de stimulation lumineuse (4), un affaiblisseur de lumière réglable (3), des miroirs (6 et 14) pour réfléchir la lumière, ainsi que des détecteurs de lumière (11 et 18), **caractérisé en ce que** le stimulateur visuel comprend une enceinte à miroirs (1) et un tube à miroirs (2) constitué de miroirs (6 et 14), dans lequel les points de réflexion des miroirs (6 et 14) forment différents angles les uns par rapport aux autres, et qui sont reliés les uns aux autres de telle façon que les faisceaux lumineux (27) se dispersent depuis le tube à miroirs (2) vers l'enceinte à miroirs (1) sans gêne, quelle que soit l'emplacement de l'objet (26) à examiner dans l'enceinte à miroirs, et **en ce que** l'extrémité du tube à miroirs (2) opposée à l'extrémité à laquelle est reliée l'enceinte à miroirs est équipée d'une source de stimulation lumineuse (4).

2. Stimulateur visuel selon la revendication 1, **caractérisé en ce qu'**il y a un ou plusieurs miroirs (6) dans l'enceinte à miroirs (1).

3. Stimulateur visuel selon la revendication 1 ou 2, **caractérisé en ce que** le miroir (6) de l'enceinte à miroir (1) est semblable à un plan.

4. Stimulateur visuel selon la revendication 1, **caractérisé en ce qu'**il y a un ou plusieurs miroirs (14) dans le tube à miroirs (2).

5. Stimulateur visuel selon les revendications 1 et 4, **caractérisé en ce que** le miroir (14) du tube à miroir (2) est semblable à un plan.

6. Stimulateur visuel selon la revendication 1, **caractérisé en ce que** la paroi extérieure (5) de l'enceinte à miroirs (1) est conçue en un matériau transparent.

7. Stimulateur visuel selon les revendications 1 et 6, **caractérisé en ce que** la paroi extérieure (5) de l'enceinte à miroirs (1) est conçue en plastique ou en verre.

8. Stimulateur visuel selon les revendications 1, 2 et 7, **caractérisé en ce qu'**il y a des ouvertures (9) entre les miroirs (6) de l'enceinte à miroirs (1).

9. Stimulateur visuel selon la revendication 1, **caractérisé en ce qu'**un affaiblisseur de lumière réglable (3) est placé entre la source de stimulation lumineuse (4) et le tube à miroirs (2).

10. Stimulateur visuel selon la revendication 1, **caractérisé en ce qu'**un affaiblisseur de lumière réglable (3) est placé entre le tube à miroirs (2) et l'enceinte à miroirs (1).

11. Stimulateur visuel selon la revendication 1, **caractérisé en ce que** plus d'une source de stimulation lumineuse (4) est dirigée vers le tube à miroirs (2).

12. Stimulateur visuel selon la revendication 1, **caractérisé en ce que** la source de stimulation lumineuse (4) est une lumière éclair.

13. Stimulateur visuel selon la revendication 1, **caractérisé en ce que** la source de stimulation visuelle (4) est recouverte d'un cache de protection métallique (15), hormis sa surface réfléchissante de lumière.

14. Stimulateur visuel selon la revendication 1, **caractérisé en ce qu'**un câble de fibre optiques (17) est relié à la source de stimulation lumineuse (4), ledit câble étant relié au détecteur de lumière (18) à partir duquel les informations sur l'intensité lumineuse peuvent être transmises au dispositif de traitement de données (21).

15. Stimulateur visuel selon la revendication 1, **caractérisé en ce que** l'enceinte à miroirs (1) comprend un ou plusieurs détecteurs de lumière (11) à partir desquels les informations sur l'intensité lumineuse peuvent être transmises au dispositif de traitement de données (21).

16. Stimulateur visuel selon les revendications 1, 14 et 15, **caractérisé en ce que** les détecteurs de lumière (11 et 8) comprennent un phototransistor et/ou une photodiode, ainsi qu'un amplificateur d'opération pour amplifier un signal lumineux.

17. Stimulateur visuel selon les revendications 1, 2, 3, 4, 5 et 13, **caractérisé en ce que** des surfaces argentées (6' et 14') des miroirs (6 et 14) du dispositif ainsi que le cache de protection métallique (15) de la source de stimulation lumineuse (4) sont galvaniquement reliés les uns aux autres.

18. Stimulateur visuel selon la revendication 1, **caractérisé en ce que** l'affaiblisseur de lumière réglable (3) est destiné à affaiblir différentes longueurs d'ondes lumineuses avec un rendement ajustable.
